# EUROPEAN PATENT APPLICATION

(11) **EP 1 944 283 A2**
(43) Date of publication of application: **16.07.2008**
(21) Application number: 08250081.0
(22) Date of filing: 09.01.2008
(51) Int. Cl.: C07C 2/08, C07C 67/08, C07C 69/24, C07C 11/02, B01J 31/08, B01J 31/10

(54) **Method for heterogeneous acid catalysts**

(30) Priority: 09.01.2007 US 879420 P
(71) Applicant: Rohm and Haas Company, Philadelphia, PA 19106-2399 (US)
(72) Inventor: Banavali, Rajiv Manohar, Rydal, Pennsylvania 19046 (US)
(74) Representative: Buckley, Guy Julian

(57) **Abstract**

A method for heterogeneous catalysis of organic reactions having at least one reactant and a product; said method comprising steps of (a) providing a catalyst comprising a gel-type acidic ion exchange resin; and (b) contacting said catalyst with said at least one reactant; wherein said organic reaction is selected from among esterification, alkene addition or isomerization, Friedel-Crafts acylation, alcohol or alkene carbonylation, nitration, and Ritter reaction.

## Description

### Background

This invention relates generally to a method for heterogeneous acid catalysis with ion exchange resins.

Use of ion exchange resins as heterogeneous catalysts is known - see, e.g., Georges Gelbard, Ind. Eng. Chem. Res. 2005, vol. 44, pp. 8468-8498. The prior art generally teaches that macroporous resins with a high crosslinker level are needed for effective catalysis. A gel-type resin catalyst is used for a Friedel-Crafts alkylation of phenol with acetone in U.S. Pat. No. 6,730,816. However, this reference advocates use of sulfone-bridged resins, and it does not teach catalysis of other organic reactions.

The problem addressed by this invention is to find an improved method for heterogeneous catalysis for a variety of organic reactions.

### Statement of Invention

The present invention is directed to a method for heterogeneous catalysis of an organic reaction having at least one reactant and a product; said method comprising steps of (a) providing a catalyst comprising a gel-type acidic ion exchange resin having 0.25% to 3% crosslinker; and (b) contacting said catalyst with said at least one reactant; wherein said organic reaction is selected from among esterification, alkene addition or isomerization, Friedel-Crafts acylation, alcohol or alkene carbonylation, nitration, and the Ritter reaction.

### Detailed Description

All percentages are weight percentages, and all temperatures are in °C, unless otherwise indicated. Weight percentages of ion exchange resin are based on dry resin. An "alkyl" group is a saturated hydrocarbyl group having from one to twenty carbon atoms in a linear, branched or cyclic arrangement. In one preferred embodiment, alkyl groups are acyclic. An "alkene" is a compound having from two to twenty carbon atoms in a linear, branched or cyclic arrangement, and having at least one carbon-carbon double bond. In one preferred embodiment, an alkene contains only carbon and hydrogen. An "aromatic" compound has from six to twenty carbon atoms and one or more rings; multiple rings may be attached or fused.

At least one reactant and the product of the organic reaction are organic compounds, i.e., compounds containing carbon and hydrogen. In one embodiment, organic compounds may contain, in addition to carbon and hydrogen, element(s) selected from nitrogen, phosphorus, oxygen, sulfur, and halogens. Preferably, organic compounds have from one to twenty carbon atoms.

In one embodiment of the invention, the reaction is an esterification reaction in which the reactants comprise an organic acid and/or anhydride and an alcohol. The organic acid may be a mono-, di- or poly-carboxylic acid, or a mixture thereof. The alcohol may be a mono-, di- or poly-hydric alcohol, and may be an aliphatic alcohol or an aromatic alcohol (e.g., phenol), or a mixture thereof. In one embodiment, the alcohol is a C₁-C₈ aliphatic alcohol or diol. The product is an ester. In one aspect of this embodiment, the organic acid is a fatty acid. Fatty acids are acyclic aliphatic carboxylic acids containing from 8 to 20 carbon atoms; typically, they contain from 12 to 18 carbon atoms. With respect to carbon-carbon bonds, the fatty acids may be saturated, monounsaturated or polyunsaturated (typically 2 or 3 carbon-carbon double bonds). Natural fats (triglycerides) are triesters of glycerin and fatty acids, and may also contain small amounts of other esterified, or free fatty acids, as well as small amounts (1-4%) of phospholipids, e.g., lecithin, and very small amounts (<1%) of other compounds, e.g., tocopherols. Preferably, the fatty acid contains less than 1% triglycerides, more preferably less than 0.5% and most preferably, it is substantially free of triglycerides. Preferably, the C₁-C₈ aliphatic alcohol or diol is a C₁-C₄ alcohol; alternatively it is methanol, ethanol or n-butanol; alternatively it is methanol or ethanol; and most preferably methanol. In one embodiment of the invention, the C₁-C₈ aliphatic alcohol or diol is a C₁-C₈ diol, alternatively a C₁-C₄ diol, e.g., ethylene glycol. In one embodiment of the invention, the acid or anhydride is maleic anhydride. In this embodiment, preferably the alcohol is methanol.

In one embodiment of the invention, the alcohol is present in an amount of at least 1.1 equivalents based on the organic acid, alternatively at least 2 equivalents, alternatively at least 5 equivalents, alternatively at least 10 equivalents, alternatively at least 15 equivalents. In one embodiment of the invention, the alcohol is present in an amount of no more than 25 equivalents.

In an esterification reaction, preferably the reaction mixture is heated in a temperature range from 40°C to 150°C for at least 15 minutes in contact with the catalyst. Alternatively, the temperature is at least 50°C, alternatively at least 55°C, alternatively at least 60°C. Alternatively, the temperature is no greater than 110°C, alternatively no greater than 90°C, alternatively no greater than 85°C, alternatively no greater than 80°C, alternatively no greater than 75°C. When the reaction is carried out in a batch reactor, preferably the reaction time is at least 0.5 hour, alternatively at least 1 hour, alternatively at least 2 hours, alternatively at least 3 hours, alternatively at least 6 hours. Alternatively, the reaction time is no greater than 24 hours, alternatively no greater than 16 hours, alternatively no greater than 10 hours, alternatively no greater than 6 hours. In an embodiment where the temperature is from 55-75°C, the reaction time is from 0.5-6 hours. The catalyst is removed from the reaction mixture by filtration, centrifugation, or any other standard method for separating solids and liquids. When the reaction is carried out in a continuous reactor, preferably the contact time is at least 30 minutes, alternatively at least 45 minutes. Preferably, the contact time is no more than 6 hours, alternatively no more than 4 hours, alternatively no more than 2 hours.

In another embodiment of the invention, the reactants comprise an alkene. In one aspect of this embodiment, the alkene undergoes an acid-catalyzed addition reaction in which the alkene either adds to another organic molecule in an electrophilic addition reaction, e.g., alkylation of an aromatic compound by a protonated alkene, or alkene dimerization or oligomerization; or another molecule adds to the alkene, e.g., water, an alcohol or a carboxylic acid. Particular examples of alkene addition reactions include alkylation of phenols with alkenes to produce alkylphenols, alkene hydrations to alcohols, alkene etherifications with alcohol, and alkene esterifications with carboxylic acids. In another aspect of this embodiment, the alkene is the sole reactant, and undergoes an acid-catalyzed alkene isomerization. In one aspect of this embodiment, additions of alkenes to phenols are excluded from the scope of the invention.

General conditions suitable for acid-catalyzed reactions in this invention include 2-10% catalyst by weight of the reaction mixture, preferably 5-10%. Suitable temperatures will vary considerably with the nature of the reactants, but can be determined from known reaction conditions by one skilled in the art.

In one embodiment of the invention, the organic reaction is a Friedel-Crafts acylation. One reactant is an aromatic organic compound, typically a hydrocarbon, ether or phenol. In one aspect of this embodiment, reactions of phenols are excluded from the scope of the invention. In another embodiment, the organic reaction is an alcohol or alkene carbonylation, including hydroformylation reactions. In another embodiment, the organic reaction is a nitration, e.g., an electrophilic reaction which produces a nitroaromatic compound. In another embodiment, the organic reaction is a Ritter reaction.

The ion exchange resin used in the present invention is a gel-type resin, not a macroreticular resin. A macroreticular resin is a resin having a surface area from 25 m²/g to 200 m²/g and an average pore diameter from 50 Å to 500 Å; alternatively a surface area from 30 m²/g to 80 m²/g and an average pore diameter from 100 Å to 300 Å. Suitable gel-type resins include, e.g., acrylic resins, styrenic resins, and combinations thereof. Resins contain polymerized units of a multiethylenically unsaturated monomer (crosslinker). Preferably, the level of crosslinker in the resin is no more than 2.75%, alternatively no more than 2.5%, alternatively no more than 2.25%, alternatively no more than 2%, alternatively no more than 1.75%. In one embodiment, the level of crosslinker is at least 0.5%, alternatively at least 0.75%, alternatively at least 1%, alternatively at least 1.25%. Preferably, the average particle size of the gel resin is from 100 µm to 2000 µm, more preferably from 200 µm to 800 µm. In one embodiment of the invention, the ion exchange resin comprises polymerized units of styrene and a crosslinker, e.g., divinyl aromatics; di-, tri- and tetra-(meth)acrylates or (meth)acrylamides; di-, tri- and tetra-allyl ethers and esters; polyallyl and polyvinyl ethers of glycols and polyols. In one embodiment of the invention, the crosslinker is diethylenically unsaturated, e.g., divinylbenzene (DVB). In one embodiment of the invention, the acid functionality of the ion exchange resin comprises sulfonic acid groups, carboxylic acid groups, phosphoric acid groups or a mixture thereof. A typical acidic ion exchange resin has from 0.4 to 8 meq/kg acid functionality, on a dry basis, alternatively at least 2 meq/kg, alternatively at least 4 meq/kg. Preferably, the acid functionality is in the form of sulfonic acid groups. In one embodiment of the invention, the resin does not contain sulfone bridging groups, i.e., sulfone cross-linking, as described in U.S. Pat. No. 6,730,816.

In one embodiment of the invention, when the reaction is carried out in a batch reactor, the resin is present in an amount from 0.1% to 20% (based on dry weight of resin) of the reaction mixture, alternatively from 1% to 15%, alternatively from 2% to 8%. The reaction also may be carried out in a continuous reactor in which the catalyst is confined to the reactor, e.g., in a catalyst bed.

### Examples

### Example 1: Esterification of Stearic Acid

In a four-neck 1L RB flask equipped with a Soxhlet condenser containing 50 g activated molecular sieves 3A, thermometer and mechanical stirrer, was added methanol rinsed 2% cross-linked gel strong acid cation ion exchange resin catalyst beads (13.75 g, 5% by weight of reaction mixture). Then, stearic acid (22.5 g; 0.079 moles) and methanol (50 g, 1.56 mole or 20 equivalent of acid) was charged to the flask and mechanical stirring started at 185 RPM. The flask was heated by external infrared lamp to reach 60 °C over 20 minutes. The mixture was allowed to reach reflux temperature (~65-67°C) with efficient stirring (235 rpm). The reflux was condensed through a water condenser and passed through the molecular sieves back into the flask.

The reaction was carried out at 65°C-67°C (reflux temperature) and atmospheric pressure for 2 hours. Samples were taken at 30 minute intervals, using long stem polyethylene pipette with small bore to avoid withdrawing catalyst beads. Samples were filtered through 0.45 µm MILLIPORE PTFE filter into a tared one ounce glass vial. Sample weight was recorded. After 2 hours, the mixture was cooled to ambient temperature. The catalyst was recovered by filtration from the organic phase. A final sample of the liquid phase was taken for analysis. GC/MS analysis of the reaction mixture was conducted to analyze for methyl stearate. The analysis showed 100 % conversion of stearic acid to methyl stearate.

### Comparative Example 1: Esterification with Highly Cross-Linked Catalysts

The esterification of Example 1 was run with a 4% cross-linked gel strong acid cation ion exchange resin catalyst, and also with an 18.5% cross-linked macroreticular strong acid cation ion exchange resin catalyst. The % yields of methyl stearate obtained in Example 1 and in this Comparative Example after two hours are tabulated below.

| Example | Catalyst¹ | HMS (mm) | wt cap (meq/g) | vol cap (eq/L) | 2 hr yield |
|---|---|---|---|---|---|
| 1 | Gel-2%DVB | 0.75 | 5.13 | 1.17 | 100% |
| Comp. 1 | Gel-4%DVB | 0.75 | 5.6 | 1.54 | 70% |
| Comp. 1 | MR-18.5%DVB | 0.82 | 5.2 | 1.9 | 70% |

| | | | | | |
|---|---|---|---|---|---|
| 1. MR=macroreticular. Catalysts had the following properties: all were styrenic resins having sulfonic acid groups. The harmonic mean size of the resin beads (HMS) and the weight capacity (wt cap) and volume capacity (vol cap) are listed. | | | | | |

### Example 2: Olefin Dimerization- Nonene dimerization to dinonene

In a three-neck 1L RB flask equipped with a condenser, bottom filter valve, mechanical stirrer, and thermometer, was added dry, 2% cross-linked gel strong acid cation ion exchange resin catalyst beads (37.5g, 12.5% by weight of reaction mixture). Then the reactor was charged with 300 g of fresh nonene. The entire system was purged with a pre-dried nitrogen stream and a slow but steady stream of nitrogen was maintained throughout the entire reaction sequence. While stirring vigorously, the reaction mixture was heated to 115 °C (±2 °C). Under these conditions, 15-20 minutes were needed to reach 115 °C. After the desired temperature was reached, samples were taken every few minutes and measured for the refractive index at 25 °C. When the refractive index of the sample reached 1.4375 (60% conversion) in 30 minutes, the reaction was stopped by turning off the heat supply and the stirring. The reaction mixture was allowed to cool to about 80 °C and then the liquid was filtered through the filter at the bottom of the reactor, by maintaining a slight positive pressure of nitrogen directly into the strip flask. In the strip step, the liquid was heated under vacuum to strip the unreacted nonene through a water condenser. The end point conditions were 125 °C at 40 mm Hg (5.3 kPa). After cooling to room temperature, the product dinonene was transferred to a storage vessel.

## Claims

1. A method for heterogeneous catalysis of an organic reaction having at least one reactant and a product; said method comprising steps of:
(a) providing a catalyst comprising a gel-type acidic ion exchange resin having 0.25% to 3% crosslinker; and
(b) contacting said catalyst with said at least one reactant;
wherein said organic reaction is selected from among esterification, alkene addition or isomerization, Friedel-Crafts acylation, alcohol or alkene carbonylation, nitration, and the Ritter reaction.

2. The method of claim 1 in which the organic reaction is an esterification; said at least one reactant comprises an organic acid, an organic anhydride, or a mixture thereof, and an alcohol; and the product is an ester.

3. The method of claim 2 in which the catalyst has from 0.5% to 2.75% crosslinker.

4. The method of claim 3 in which said at least one reactant comprises a fatty acid having less than 1% triglyceride content and a C₁-C₈ aliphatic alcohol or diol.

5. The method of claim 4 in which the ion exchange resin has sulfonic acid functionality, and does not have sulfone bridging groups.

6. The method of claim 3 in which said at least one reactant comprises an organic anhydride and a C₁-C₈ aliphatic alcohol or diol.

7. The method of claim 1 in which said at least one reactant comprises an alkene.

8. The method of claim 1 in which the organic reaction is a Friedel-Crafts acylation.

9. The method of claim 1 in which the organic reaction is an alcohol or alkene carbonylation.

10. The method of claim 1 in which the organic reaction is a nitration.
